# EUROPEAN PATENT SPECIFICATION

(11) **EP 2 635 216 B1**
(45) Date of publication and mention of the grant of the patent: **01.09.2021**
(21) Application number: 11784883.8
(22) Date of filing: 04.11.2011
(51) Int. Cl.: A61B 18/00, A61B 17/32, A61B 18/14

(54) **USER FEEDBACK THROUGH HANDPIECE OF SURGICAL INSTRUMENT**
BENUTZERFEEDBACK ÜBER EIN HANDSTÜCK EINES CHIRURGISCHENINSTRUMENTS
RÉTROACTION D'UTILISATEUR PAR PIÈCE À MAIN D'INSTRUMENT CHIRURGICAL

(30) Priority: 19.10.2011 US 201113276660; 05.11.2010 US 410603 P; 19.05.2011 US 201161487846 P
(43) Date of publication of application: 11.09.2013
(73) Proprietor: Cilag GmbH International, 6300 Zug (CH)
(72) Inventor: HOUSER, Kevin, L., Springboro OH 45066 (US); DANNAHER, William, D., Cincinnati OH 45236 (US); DIETZ, Timothy, G., Wayne, PA 19087 (US); STULEN, Foster, B., Mason OH 45040 (US); SHELTON, Frederick, E., IV, Hillsboro OH 45133 (US); YATES, David, C., West Chester OH 45069 (US); WORRELL, Barry, C., Centerville OH 45458 (US)
(74) Representative: Tunstall, Christopher Stephen
(86) International application number: PCT/US2011/059358
(87) International publication number: WO 2012/061722

(56) References cited:
- EP-A1- 2 345 454
- WO-A1-01/91100
- WO-A1-2010/096174
- WO-A2-2007/024983
- WO-A2-2010/030850
- GB-A- 2 440 566
- US-A1- 2004 116 952
- US-A1- 2010 004 669
- US-A1- 2010 016 855
- US-A1- 2010 036 405
- US-A1- 2010 179 587

## Description

### BACKGROUND

In some settings, endoscopic surgical instruments may be preferred over traditional open surgical devices since a smaller incision may reduce the post-operative recovery time and complications. Consequently, some endoscopic surgical instruments may be suitable for placement of a distal end effector at a desired surgical site through a cannula of a trocar. These distal end effectors may engage tissue in a number of ways to achieve a diagnostic or therapeutic effect (e.g., endocutter, grasper, cutter, stapler, clip applier, access device, drug/gene therapy delivery device, and energy delivery device using ultrasound, RF, laser, etc.). Endoscopic surgical instruments may include a shaft between the end effector and a handle portion, which is manipulated by the clinician. Such a shaft may enable insertion to a desired depth and rotation about the longitudinal axis of the shaft, thereby facilitating positioning of the end effector within the patient.

Examples of endoscopic surgical instruments include those disclosed in U.S. Pat. Pub. No. 2006/0079874, entitled "Tissue Pad Use with an Ultrasonic Surgical Instrument," published April 13, 2006; U.S. Pat. Pub. No. 2007/0191713, entitled "Ultrasonic Device for Cutting and Coagulating," published August 16, 2007; U.S. Pat. Pub. No. 2007/0282333, entitled "Ultrasonic Waveguide and Blade," published December 6, 2007; U.S. Pat. Pub. No. 2008/0200940, entitled "Ultrasonic Device for Cutting and Coagulating," published August 21, 2008; U.S. Pub. No. 2009/0209990 entitled "Motorized Surgical Cutting and Fastening Instrument Having Handle Based Power Source," published August 20, 2009; and U.S. Pub. No. 2010/0069940 entitled "Ultrasonic Device for Fingertip Control," published March 18, 2010; U.S. Pat. Pub. No. 2011/0015660, entitled "Rotating Transducer Mount for Ultrasonic Surgical Instruments," published January 20, 2011; U.S. Pat. No. 6,500,176, entitled "Electrosurgical Systems and Techniques for Sealing Tissue," issued December 31, 2002; U.S. Pat. No. 7,416,101 entitled "Motor-Driven Surgical Cutting and Fastening Instrument with Loading Force Feedback," issued August 26, 2008; U.S. Pat. No. 7,738,971 entitled "Post-Sterilization Programming of Surgical Instruments," issued June 15, 2010; and U.S. Pat. Pub. No. 2011/0087218, entitled "Surgical Instrument Comprising First and Second Drive Systems Actuatable by a Common Trigger Mechanism," published April 14, 2011. Additionally, such surgical tools may include a cordless transducer such as that disclosed in U.S. Pat. Pub. No. 2009/0143797, entitled "Cordless Hand-held Ultrasonic Cautery Cutting Device," published June 4, 2009. In addition, the surgical instruments may be used, or adapted for use, in robotic-assisted surgery settings such as that disclosed in U.S. Pat. No. 6,783,524, entitled "Robotic Surgical Tool with Ultrasound Cauterizing and Cutting Instrument," issued August 31, 2004.

US2010/016855 (A1) discloses a modular tool comprising a handle portion and a distal portion. The handle portion is configured to be manipulated by a user. The distal portion is configured to be attached to the handle portion, but is further configured to be removable from the handle portion by the user. Manipulation of the handle portion causes movement of one or more components of the distal portion. The distal portion is further configured to sense one or more parameters and transmit the sensed parameters to the handle portion. The handle portion may include a haptic actuator.

US2010/036405 (A1) discloses an apparatus, system, and method for driving an end effector coupled to an ultrasonic transducer in a surgical instrument. Feedback may be provided by an output indicator. An output indicator may provide visual, audible, and/or tactile feedback to alert the user of the ultrasonic surgical instrument that a change in tissue state has occurred.

WO 2010/030850 A2 discloses an ultrasonic surgical instrument comprising a handpiece and an ultrasonically actuated blade distal to the handpiece. The instrument includes an activation member that is operable to selectively activate the blade and a controller that is operable to select the energy level at which the blade will be activated. In one example, a rocker switch provides haptic feedback to a user when either a distal portion or a proximal portion of the rocker switch has been sufficiently depressed, providing confirmation to a user that the blade has been activated.

While several systems and methods have been made and used for surgical instruments, it is believed that no one prior to the inventors has made or used the invention described in the appended claims.

The present invention provides a surgical instrument, as defined in claim 1. Further optional features of the invention are defined in the dependent claims.

### BRIEF DESCRIPTION OF THE DRAWINGS

While the specification concludes with claims which define the present invention and which particularly point out and distinctly claim this technology, it is believed this technology will be better understood from the following description of certain examples taken in conjunction with the accompanying drawings, in which like reference numerals identify the same elements and in which:
FIG. 1 depicts a schematic view of a medical device, which is not in accordance with the present invention, having an internal power source;
FIG. 2 depicts a perspective view of a medical device, which is not in accordance with the present invention, having an internal power source;
FIG. 3 depicts a side elevational view of an exemplary battery powered surgical instrument having a circuit board and a haptic indicator;
FIG. 4 depicts a side elevational view of an exemplary embodiment of a surgical instrument of the present invention having a feedback pad;
FIG. 5 depicts a side elevational view of a surgical instrument, which is not in accordance with the present invention, including a display screen;
FIG. 6 depicts a spring loaded gauge in communication with a trigger of the instrument of FIG. 5;
FIG. 7 depicts a pressure sensor in communication with a trigger of the instrument of FIG. 5;
FIG. 8 depicts a side elevational view of a surgical instrument, which is not in accordance with the present invention, including a display screen as a visual indicator and speaker holes as an auditory indicator;
FIG. 9 depicts a plan view of a top surface of the surgical instrument of FIG. 8;
FIG. 10 depicts a top plan view of a surgical instrument, which is not in accordance with the present invention, including a visual indicator screen;
FIG. 11 depicts a cross-sectional view of the instrument of FIG. 10, showing a path for transmission of emitted light from lighting devices to the visual indicator screen;
FIG. 12 depicts a cross-sectional view of the instrument of FIG. 10, showing an alternate path for transmission of emitted light from lighting devices to the visual indicator screen;
FIG. 13 depicts a cross-sectional view of the instrument of FIG. 10, showing yet another path for transmission of emitted light from lighting devices to the visual indicator screen; and
FIG. 14, which is not in accordance with the present invention, depicts a cross-sectional view of an alternate version of the surgical instrument of FIG. 13, in which the flexible display is replaced with a projecting lens.

The drawings are not intended to be limiting in any way, and it is contemplated that various embodiments of the technology may be carried out in a variety of other ways, within the scope of claim 1, including those not necessarily depicted in the drawings. The accompanying drawings incorporated in and forming a part of the specification illustrate several aspects of the present technology, and together with the description serve to explain the principles of the technology; it being understood, however, that this technology is not limited to the precise arrangements shown.

### DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the scope of appended claim 1. Accordingly, the drawings and descriptions should be regarded as illustrative in nature and not restrictive.

### I. Medical Devices for Use With Insertable or Reclaimable Components

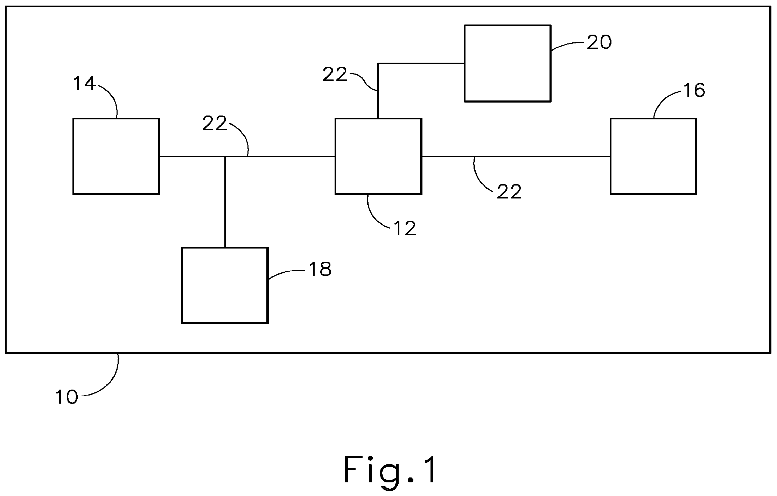

FIG. 1 shows components of a medical device (10) in diagrammatic block form. As shown, medical device (10) comprises a control module (12), a power source (14), and an end effector (16). Merely exemplary power sources (14) may include NiMH batteries, Li-ion batteries (e.g., prismatic cell type lithium ion batteries, etc.), Ni-Cad batteries, or any other type of power source as may be apparent to one of ordinary skill in the art in light of the teachings herein. Control module (12) may comprise a microprocessor, an application specific integrated circuit (ASIC), memory, a printed circuit board (PCB), a storage device (such as a solid state drive or hard disk), firmware, software, or any other suitable control module components as will be apparent to one of ordinary skill in the art in light of the teachings herein. Control module (12) and power source (14) are coupled by an electrical connection (22), such as a cable and/or traces in a circuit board, etc., to transfer power from power source (14) to control module (12). Alternatively, power source (14) may be selectively coupled to control module (12). This allows power source (14) to be detached and removed from medical device (10), which may further allow power source (14) to be readily recharged or reclaimed for resterilization and reuse, such as in accordance with the various teachings herein. In addition or in the alternative, control module (12) may be removed for servicing, testing, replacement, or any other purpose as will be apparent to one of ordinary skill in the art in view of the teachings herein.

End effector (16) is coupled to control module (12) by another electrical connection (22). End effector (16) is configured to perform a desired function of medical device (10). By way of example only, such function may include cauterizing tissue, ablating tissue, severing tissue, ultrasonically vibrating, stapling tissue, or any other desired task for medical device (10). End effector (16) may thus include an active feature such as an ultrasonic blade, a pair of clamping jaws, a sharp knife, a staple driving assembly, a monopolar RF electrode, a pair of bipolar RF electrodes, a thermal heating element, and/or various other components. End effector (16) may also be removable from medical device (10) for servicing, testing, replacement, or any other purpose as will be apparent to one of ordinary skill in the art in view of the teachings herein. In some versions, end effector (16) is modular such that medical device (10) may be used with different kinds of end effectors (e.g., as taught in U.S. Provisional Application Serial No. 61/410,603, etc.). Various other configurations of end effector (16) may be provided for a variety of different functions depending upon the purpose of medical device (10) as will be apparent to those of ordinary skill in the art in view of the teachings herein. Similarly, other types of components of a medical device (10) that may receive power from power source (14) will be apparent to those of ordinary skill in the art in view of the teachings herein.

Medical device (10) of the present example includes a trigger (18) and a sensor (20), though it should be understood that such components are merely optional. Trigger (18) is coupled to control module (12) and power source (14) by electrical connection (22). Trigger (18) may be configured to selectively provide power from power source (14) to end effector (16) (and/or to some other component of medical device (10)) to activate medical device (10) when performing a procedure. Sensor (20) is also coupled to control module (12) by an electrical connection (22) and may be configured to provide a variety of information to control module (12) during a procedure. By way of example only, such configurations may include sensing a temperature at end effector (16) or determining the oscillation rate of end effector (16). Data from sensor (20) may be processed by control module (12) to effect the delivery of power to end effector (16) (e.g., in a feedback loop, etc.). Various other configurations of sensor (20) may be provided depending upon the purpose of medical device (10) as will be apparent to those of ordinary skill in the art in view of the teachings herein. Of course, as with other components described herein, medical device (10) may have more than one sensor (20), or sensor (20) may simply be omitted if desired.

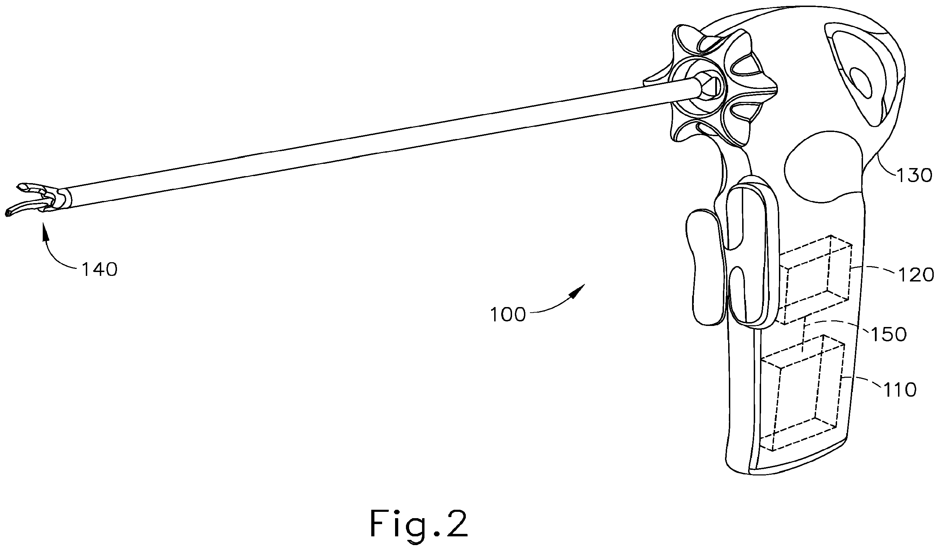

FIG. 2 depicts a merely exemplary form that medical device (10) may take. In particular, FIG. 2 shows a medical device (100) comprising a power source (110), a control module (120), a housing (130), end effector (140), and an electrical connection (150). In the present example, power source (110) is located internally within housing (130) of medical device (100). Alternatively, power source (110) may only partially extend into housing (130) and may be selectively attachable to a portion of housing (130). In yet a further exemplary configuration, a portion of housing (130) may extend into power source (110) and power source (110) may be selectively attachable to the portion of housing (130). Power source (110) may also be configured to detach from medical device (100) and decouple from control module (120) or electrical connection (150). As a result, power source (110) may be completely separated from medical device (100) in some versions. As is readily apparent, this may allow the power source (110) to be removed to be recharged or reclaimed for resterilization and reuse, such as in accordance with various teachings herein. After recharging, or after an initial charge, power source (110) may be inserted or reinserted into medical device (100) and secured to housing (130) or internally within housing (130). Of course, medical device (100) may also allow power source (110) to be charged and/or recharged while power source (110) is still in or otherwise coupled relative to housing (130).

It should also be understood that control module (120) may be removed for servicing, testing, replacement, or any other purpose as will be apparent to one of ordinary skill in the art in view of the teachings herein. Further, end effector (140) may also be removable from medical device (100) for servicing, testing, replacement, or any other purpose as will be apparent to one of ordinary skill in the art in view of the teachings herein. In some versions, power source (110) is external to medical device (100). For instance, power source (110) may be constructed in accordance with at least some of the teachings of U.S. Pat. Pub. No. 2011/0087212, entitled "Surgical Generator for Ultrasonic and Electrosurgical Devices," published April 14, 2011.

While certain configurations of an exemplary medical device (100) have been described, various other ways in which medical device (100) may be configured will be apparent to those of ordinary skill in the art in view of the teachings herein. By way of example only, medical devices (10, 100) and/or any other medical device referred to herein may be constructed in accordance with at least some of the teachings of U.S. Pat. No. 5,322,055 entitled "Clamp Coagulator/Cutting System for Ultrasonic Surgical Instruments," issued June 21, 1994; U.S. Pat. No. 5,873,873 entitled "Ultrasonic Clamp Coagulator Apparatus Having Improved Clamp Mechanism," issued February 23, 1999; U.S. Pat. No. 5,980,510, entitled "Ultrasonic Clamp Coagulator Apparatus Having Improved Clamp Arm Pivot Mount," filed October 10, 1997; U.S. Pat. No. 6,325,811 entitled "Blades with Functional Balance Asymmetries for use with Ultrasonic Surgical Instruments," issued December 4, 2001; U.S. Pub. No. 2006/0079874 entitled "Tissue Pad for Use with an Ultrasonic Surgical Instrument," published April 13, 2006,; U.S. Pub. No. 2007/0191713 entitled "Ultrasonic Device for Cutting and Coagulating," published August 16, 2007; U.S. Pub. No. 2007/0282333 entitled "Ultrasonic Waveguide and Blade," published December 6, 2007; U.S. Pub. No. 2008/0200940 entitled "Ultrasonic Device for Cutting and Coagulating," published August 21, 2008; U.S. Pub. No. 2009/0143797, entitled "Cordless Hand-held Ultrasonic Cautery Cutting Device," published June 4, 2009; U.S. Pub. No. 2010/0069940 entitled "Ultrasonic Device for Fingertip Control," published March 18, 2010; U.S. Pub. No. 2011/0015660, entitled "Rotating Transducer Mount for Ultrasonic Surgical Instruments," published January 20, 2011; and/or U.S. Provisional Application Serial No. 61/410,603, filed November 5, 2010, entitled "Energy-Based Surgical Instruments".

Of course, housing (130) and medical device (100) may include other configurations. For instance, housing (130) and/or medical device (100) may include a tissue cutting element and one or more elements that transmit bipolar RF energy to tissue (e.g., to coagulate or seal the tissue). An example of such a device is the ENSEAL® Tissue Sealing Device by Ethicon Endo-Surgery, Inc., of Cincinnati, Ohio. Further examples of such devices and related concepts are disclosed in U.S. Pat. No. 6,500,176 entitled "Electrosurgical Systems and Techniques for Sealing Tissue," issued December 31, 2002; U.S. Pat. No. 7,112,201, entitled "Electrosurgical Instrument and Method of Use," issued September 26, 2006; U.S. Pat. No. 7,125,409, entitled "Electrosurgical Working End for Controlled Energy Delivery," issued October 24, 2006; U.S. Pat. No. 7,169,146 entitled "Electrosurgical Probe and Method of Use," issued January 30, 2007; U.S. Pat. No. 7,186,253, entitled "Electrosurgical Jaw Structure for Controlled Energy Delivery," issued March 6, 2007; U.S. Pat. No. 7,189,233, entitled "Electrosurgical Instrument," issued March 13, 2007; U.S. Pat. No. 7,220,951, entitled "Surgical Sealing Surfaces and Methods of Use," issued May 22, 2007; U.S. Pat. No. 7,309,849, entitled "Polymer Compositions Exhibiting a PTC Property and Methods of Fabrication," issued December 18, 2007; U.S. Pat. No. 7,311,709, entitled "Electrosurgical Instrument and Method of Use," issued December 25, 2007; U.S. Pat. No. 7,354,440, entitled "Electrosurgical Instrument and Method of Use," issued April 8, 2008; U.S. Pat. No. 7,381,209, entitled "Electrosurgical Instrument," issued June 3, 2008; and U.S. Pub. No. 2011/0087218, entitled "Surgical Instrument Comprising First and Second Drive Systems Actuatable by a Common Trigger Mechanism," published April 14, 2011.

### II. Exemplary Haptic or Visual Indicators on Ultrasonic Surgical Instrument

Examples described below relate to uses of haptic and/or visual information indicating devices with surgical instruments. It should be understood that the teachings below may be readily incorporated into any of the devices (10, 100) described above and/or any of the devices described in the various references cited herein. Throughout this disclosure, reference numbers utilized with different alphanumeric extensions indicate similar components in different versions of a described reference (i.e., waveguide (210) and waveguide (210F)).

### A. Exemplary Haptic Indicators

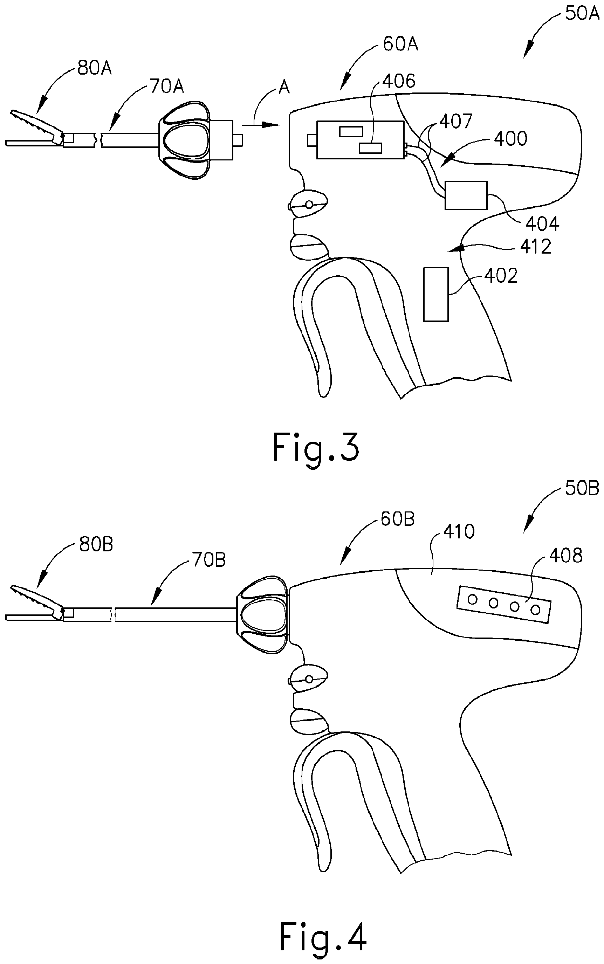

FIG. 3 shows an exemplary surgical instrument (50A), similar to medical device (10, 100) described above, that additionally includes a haptic user feedback system (400). Haptic indicators are currently used in application for cell phones, gaming devices, and other similar applications known to those of ordinary skill in the art. Such devices with haptic indicators provide a tactile sensation to provide a user with feedback. For example, such devices may cause a perceptible continuation or sporadic vibration to a user (to indicate, for example, an incoming call on a cell phone); other devices may provide another type of impulse in reaction to a triggering event or change in status of certain received data or information.

Instrument (50A) includes end effector (80A), multi-piece handle assembly (60A), and elongated transmission assembly (70A). Transmission assembly (70A) may be removably received in the direction of arrow (A) within a distal aperture (not shown) formed in a distal end of multi-piece handle assembly (60A). Instrument (50A) may additionally be a battery-powered device. Battery (402), haptic indicator (404), and circuit board (406) are housed in multi-piece handle assembly (60A).

Haptic indicator (404) may take the form of a vibration generator, for example, that may be constructed and operable in accordance with the teachings of U.S. Patent App. Publ. No. 2011/0152901, entitled "Implantable Port with Vibratory Feedback", published June 23, 2011. The vibration generator may comprise a reciprocating or oscillating weight that may be provided by a permanent magnet suspended by a resilient member. Vibration occurs as a selectively energized ring or coil causes the permanent magnet weight to reciprocate or oscillate. Other merely illustrative components and configurations of such a reciprocating weight type of a vibration generator are disclosed in U.S. Pat. No. 7,292,227, entitled "Electronic Device, Vibration Generator, Vibration-Type Reporting Method, and Report Control Method," issued Nov. 6, 2007. Additional exemplary components and configurations that a vibration generator may incorporate are disclosed in U.S. Pat. No. 6,982,696, entitled "Moving Magnet Actuator for Providing Haptic Feedback," issued Jan. 3, 2006; and U.S. Pat. No. 5,800,336, entitled "Advanced Designs of Floating Mass Transducers," issued Sep. 1, 1998. In some versions, the vibration generator may comprise a rotatable weight located eccentric to an axis of rotation and a rotatable by a motor. The rotation of the eccentric weight may cause a vibration. Merely illustrative components and configurations that such a rotating eccentric weight type of a vibration generator may incorporate are also disclosed in U.S. Pat. No. 7,292,227. As another merely illustrative example, a rotating eccentric weight type of a vibration generator may be configured in accordance with the teachings of U.S. Pat. No. 5,107,155, entitled "Vibrator Motor for Wireless Silent Alerting Device," issued Apr. 21, 1992. By way of example only, the motor may rotate the weight at speeds up to approximately 10,000 rpm or at any other suitable speed.

As another merely illustrative example, a vibration generator may comprise a piezoelectric element. By way of example only, the vibration generator may be configured in accordance with the teachings of U.S. Pat. No. 5,277,694, entitled "Electromechanical Transducer for Implantable Hearing Aids," issued Jan. 11, 1994. As another example, the vibration generator may be configured in accordance with the teachings of U.S. Pat. No. 7,583,564, entitled "Piezoelectric Actuator and Electronic Equipment with Piezoelectric Actuator," issued Sep. 1, 2009. In some other versions, the vibration generator may comprise a piezoelectric element that is contained within a clamshell housing. When the piezoelectric element is excited (e.g., by a transcutaneously applied field, by a signal from a wire that is coupled with the piezoelectric element, etc.), the outward motion of the piezoelectric element pushes outwardly on each half of the surrounding clamshell housing. A vibratory mass may be coupled with one of the clamshell halves, while the other clamshell half may be "grounded" or secured directly to a medical device such as instrument (50A). Thus, if the piezoelectric element pushes upwardly a distance "x" and downwardly a distance "x," the displacement of the vibratory mass relative to the "ground" of instrument (50A) would be "2x."

As yet another merely illustrative example, the vibration generator may comprise a magnetostrictive material. For instance, a magnetostrictive material may be provided as a coil that is wrapped around an inner shaft. The inner shaft may contain the exciting element that acts on the magnetostrictive material. For instance, the inner shaft may be formed by an electromagnet that is selectively activated at a frequency selected to provide a rapid expansion and contraction of the magnetorestrictive material. In particular, when the magnetostrictive material is selectively excited by the inner shaft, the magnetorestrictive material expands and contracts at a rapid rate, producing vibration as it hits upper and lower bounding diaphragms.

As another merely illustrative example, the vibration generator may comprise a lever arm that has a mass at one end and a piezoelectric element under the other end. The piezoelectric element and the fulcrum support of the lever arm may be "grounded" or secured directly to a medical device such as instrument (50A). Other forms of the vibration generator may include one or more solenoids, rotational motors, steppers, etc. In some versions, a vibratory actuator within a vibration generator acts a corporeal vibration/motion energy harvester. For instance, such an energy harvesting actuator may act as an actuator "run in reverse" while it is not actively vibrating instrument (50A), to passively collect energy that can be stored in a battery or capacitor within instrument (50A) for later use when the vibration generator needs to be activated to vibrate. Various ways in which a vibratory actuator (or other component) within the vibration generator or elsewhere can be configured to act as such an energy harvester will be apparent to those of ordinary skill in the art in view of the teachings herein. It should therefore be understood that the vibration generator may be powered by an implanted power source, by an external coil, and/or by an implanted energy harvester, including combinations thereof. Similarly, and regardless of whether an energy harvester is included, other suitable variations, components, features, and configurations of a vibration generator will be apparent to those of ordinary skill in the art in view of the teachings herein.

It should be understood that the choice of the mass of instrument (50A) and the choice of the mass of a moving member in a vibration generator may determine a resonant frequency of the vibration generator. For instance, such masses and resultant resonant frequencies may be selected in accordance with the teachings of Dietz, et al.; "Partially Implantable Vibrating Ossicular Prosthesis"; Transducers '97; International Conference on Solid State Sensors and Actuators; Chicago, Jun. 16-19, 1997; Vol. 1., pp. 433-436. Suitable resonant frequencies for the vibration generator, as well as methods/equations for determining a resonant frequency of a vibration generator, will thus be apparent to those of ordinary skill in the art. In addition, it should be understood that the vibration generator may generate vibrations having any suitable amplitude and frequency. By way of example only, the vibration generated by a vibration generator may have an amplitude of approximately 100 micrometers within a frequency range of approximately 1 Hz and approximately 100 Hz. As another merely illustrative example, the vibration generated by a vibration generator may have an amplitude between approximately 100 micrometers and approximately 10 micrometers within a frequency range of approximately 5 Hz and approximately 100 Hz. As yet another merely illustrative example, the vibration generated by a vibration generator may have an amplitude between approximately 1000 micrometers and approximately 1 micrometer within a frequency range of approximately 10 Hz and approximately 800 Hz. Still other various suitable vibratory frequencies and amplitudes that may be generated by a vibration generator will be apparent to those of ordinary skill in the art in view of the teachings herein.

Circuit board (406) may be a wireless communications board to transmit wireless information about instrument (50A) in a manner apparent to those of ordinary skill in the art in view of the teachings herein. Circuit board (406) may electrically communicate with end effector (80A) via one or more electrical connections and additionally communicates with haptic indicator (404) via one or more electrical connections such as wires (407). In the present example, haptic indicator (404) is attached to pistol grip portion (412) of multi-piece handle assembly (60A). Pistol grip portion (412) encompasses an area where a user's palm will directly contact instrument (50A), such that information may be indicated to the user's palm via haptic contact with pistol grip portion (412) and closely positioned haptic indicator (404). Haptic indicator (404) may be vibrate multi-piece handle assembly (60A) while mechanical and/or electrical energy is applied to instrument (50A). Information that is communicated through haptic indicator (404) include, but are not limited to, confirmation of an activation of the end effector, a confirmation of reaching a set threshold amount of resistance measured in ohms applied across operated upon tissue that may additionally serve as a confirmation of the ending stage of severing and sealing tissue via instrument (50A) (or for a surgical instrument receiving RF energy, an indication of tissue state sensed by electrodes positioned in a respective end effector energizing such tissue). The haptic indicator may further communicate confirmation of an attachment of a removable end effector (not shown) within reusable multi-piece handle assembly (60A), an indication of lower battery power within instrument (50A), or other fault indication with respect to instrument (50A), etc. Thus, the user may be informed of important events during the surgical procedure without looking at instrument (50A) to retrieve the information, which might otherwise require the user to look away from the surgical site being operated upon.

As shown in FIG. 4, an embodiment of the surgical instrument (50B) of the present invention includes user feedback pad (408) on an exterior, proximal upper surface (410) for a user to activate and/or operate via the user's fingers. The user feedback pad (408) and haptic indicator (404) form a single unit. FIG. 4 also shows transmission assembly (70B) permanently attached to multi-piece handle assembly (60B), though it should be understood that transmission assembly (70B) may be removable from handle assembly (60B) in some versions. During use of instrument (50B), a user interacts with user feedback pad (408) to obtain information and to input settings for instrument (50B). To ensure that a user has feedback indicating that instrument (50B) has accepted user entered new settings, for example, haptic indicator (404) may provide a tactile feedback such as a vibration. Haptic feedback may provide direct feedback for button pushes and may also provide an indication that instrument (50B) is active while providing an indication of an error codes when device (50B) encounters an error. Additionally, feedback to a user indicating that instrument (50B) has accepted new user entered settings or other information may be provided in visual and/or auditory form.

Feedback systems utilized by haptic user feedback system (400) may be, for example, TouchSense® Tactile Feedback Systems and/or TouchSense® Force Feedback Systems provided by Immersion Corporation of San Jose, CA (where TouchSense® is a registered trademark of Immersion Corporation). Through use of such haptic technology, it may be possible to provide a haptic click upon completion of an insertion of transmission assembly (70A) into instrument (50A) and/or upon achieving a desirable rotation angle of end effector (80A) to signal to a user that instrument (50A) is properly assembled. For example, a torque sensor may determine when an ultrasonic blade of end effector (80A) and transmission assembly (70A) is properly attached to multi-piece handle assembly (60A) and provide a tactile "click" to a user to indicate such attachment. Also, for surgical instruments requiring one or more electrical connections, a feedback tactile "click" may be sent to a user once a required electrical connection is made.

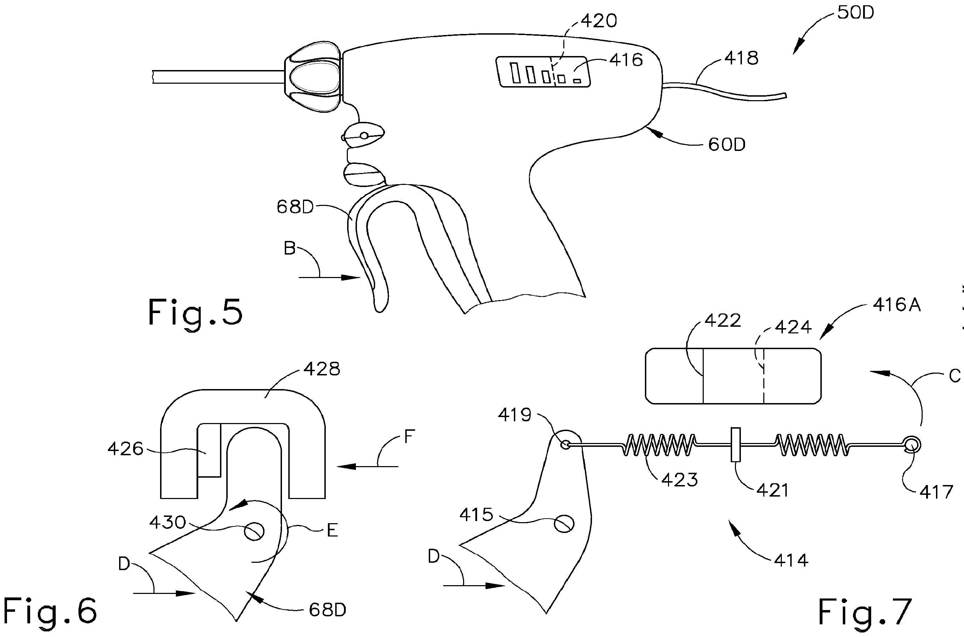

FIG. 5 shows an ultrasonic surgical instrument (50D), which is not in accordance with the present invention, and which is similar to medical device (10, 100) described above and includes a force sensor. The force sensor may comprise, for example, pressure sensor (426) of FIG. 6 or spring loaded gauge (414) of FIG. 7, both described further below. The force sensor assists with determining an amount of force a user is applying to trigger (68D) of surgical instrument (50D) in the direction arrow (B). Additionally, instrument (50D) includes a clamping end effector (e.g., like end effector (140) described above, etc.) that clamps upon application of a pressing force on trigger (68D), as described below. Further, instrument (50D) includes a visual and/or haptic indicator to indicate when a clamping force is being applied to tissue via the end effector's response to an actuation of trigger (68D). Visual user feedback panel (416) on surgical instrument (50D) may give a user a graphical representation of such an applied force via utilization of bars or other graphical indications. Additionally or alternatively, a haptic indicator, similar to haptic indicator (404) of surgical instrument (50A), may increase a frequency or level of vibration that is supplied to multi-piece handle assembly (60D). In an alternative version, a circuit board, similar to circuit board (406) of surgical instrument (50A) described above, may communicate information to visual user feedback panel (416) and/or the haptic indicator to respectively allow feedback panel (416) and/or the haptic indicator to indicate to a user the amount of power being supplied to the tissue (such power may be supplied to instrument (50D) via an electrical source, such as cable (418) connected to a generator (not shown)).

In use, a force applied by a user on trigger (68D) that effects clamping on tissue via an end effector of instrument (50D) may have beneficial or detrimental effects on the welding of the clamped tissue. A visual supporting data set, such as via feedback panel (416) described above, may be beneficial to a user, particularly in addition to an optional tonal feedback given to the user. A user may then understand what information instrument (50D) is conveying to the user within minimal disruption from the surgical procedure. Such visual representation of a user load upon instrument (50D) shown via feedback panel (416) compared to a depicted "ideal operation range" or threshold shown on feedback panel (416) via phantom lines (420) would assist a user with understanding what the status of the operated upon tissue might be. Feedback panel (416) may utilize an electrical liquid crystal display ("LCD") type of display or even light-emitted diodes ("LEDs") that light up in discrete steps areas of feedback panel (416). Types of LED displays described herein may also be used in combination with LCD types of displays as will be apparent to those of ordinary skill in the art in view of the teachings herein.

FIG. 6 shows an example of how the clamping force of the end effector of instrument (50D) may be sensed. In particular, FIG. 6 shows pressure sensor (426) built into clamp mechanism (428) of multi-piece handle assembly (60D). Pressure sensor (426) may comprise an electronic pressure sensor, or pressure transducer, converting pressure into an analog electrical signal. Such pressure transducers may utilize force collectors such as a diaphragm to measure strain or deflection due to an applied force over a space. Force collector types may include but not be limited to a piezoresistive strain gauge, capacitive strain gauge, electromagnetic strain gauge, piezoelectric strain gauge, and/or optical strain gauge. Various suitable forms that such gauges may take will be apparent to those of ordinary skill in the art in view of the teachings herein. Similarly, various suitable ways in which such strain gauges may be incorporated into clamp mechanism (428) will be apparent to those of ordinary skill in the art in view of the teachings herein.

It should be understood that sensor (426) may take a variety of additional or alternative forms. For instance, sensor (426) may be operable to measure the acoustic impedance of instrument (50D) (e.g., by comparing current to voltage). In addition or in the alternative, sensor (426) may be operable to measure electrical impedance of tissue. Furthermore, sensor (426) may comprise a displacement measuring device giving feedback on a position of a clamp arm of the end effector (not shown) (e.g., indicating whether the clamp arm is in an open position, closed position, or somewhere between). Sensor (426) may also comprise one or more thermal sensors disposed within the clamp arm of the end effector to register a clamp arm temperature and/or a tissue temperature. Sensor (426) may also comprise a pressure sensor disposed in the clamp arm of the end effector to measure the pressure applied to tissue by the clamp arm and an opposing blade of the end effector. Sensor (426) may additionally be a combination of two or more of the above-described sensors. For example, one or more sensors (426) may be operable to provide information regarding both clamp force as well as clamp arm position. Other suitable forms that sensor (426) may take will be apparent to those of ordinary skill in the art in view of the teachings herein.

Clamp mechanism (428) is coupled with the end effector (not shown) of instrument (50D) such that clamp mechanism (428), along with other components, converts pivotal movement of actuation trigger (68D) into clamping action at the end effector. In particular, clamp mechanism (428) is configured to receive a portion of actuation trigger (68D), which pivots about pin (430). As a user applies force to actuation trigger (68D) in the direction of arrow (D), actuation trigger (68D) rotates about pin (430) in the direction of arrow (E) to apply a force in the direction of arrow (F) against pressure sensor (426). Feedback from pressure sensor (426) may be sent to feedback panel (416) of surgical instrument (50D) to display the feedback information to a user via the LCD display of feedback panel (416). Feedback from pressure sensor (426) may additionally or alternatively be sent through surgical instrument (50D) as a haptic indication, as described above, and/or an audible indication, as described in greater detail below.

FIG. 7 shows another example of how the clamping force of the end effector of instrument (50D) may be sensed. A mechanical indication may occur via spring loaded gauge (414) shown in FIG. 7. Spring loaded gauge (414) of this example includes a gauge (421) positioned between a pair of opposing resilient members such as springs (423), which are connected at a fixed end to fixed point (417) in instrument (50D) and at another end to pin (419) of trigger (68D). Gauge (421) may have any of the configurations noted above for sensor (426) and/or any other suitable configuration. Spring loaded gauge (414) may measure a displacement caused by trigger (68D) and act as a transducer to convert the mechanical displacement into an electrical signal in a manner as apparent to those of ordinary skill in the art in view the teachings herein. Spring loaded gauge (414) may be placed in series with actuation trigger (68D) of surgical instrument (50D) to measure an applied force, shown via solid line (422) in FIG. 7, and transmit the measurement, as shown via arrow (C), to a panel such as feedback panel (416A) for display. For example, a user may press actuation trigger (68D) in the direction of arrow (D) such that trigger (68D) rotates about pin (415). Actuation of trigger (68D) may thus cause an expansion of spring loaded gauge (414), from which a measured of applied force may be retrieved; and release of trigger (68D) may cause spring loaded gauge (414) to return to an original, compressed position. The applied force may then be measured and compared against a preset ideal force, which is indicated as phantom line (424). A "too little force" or a "too much force" condition may thus be indicated to a user. Gauge (414) may additionally or alternatively be a solenoid type gauge. With such a gauge, an increasing force causes an increasing displacement of a core of the solenoid that can be measured in the electrical inputs to the solenoid. Gauge (414) may additionally or alternatively comprise linear variable differential transformers ("LVDTs"). Additionally or alternatively, outputs of instrument (50D) such as blade amplitude and power may be augmented by the information received from feedback features, as described above, as well as other types of indicators such as visual, haptic, and/or auditory indicators.

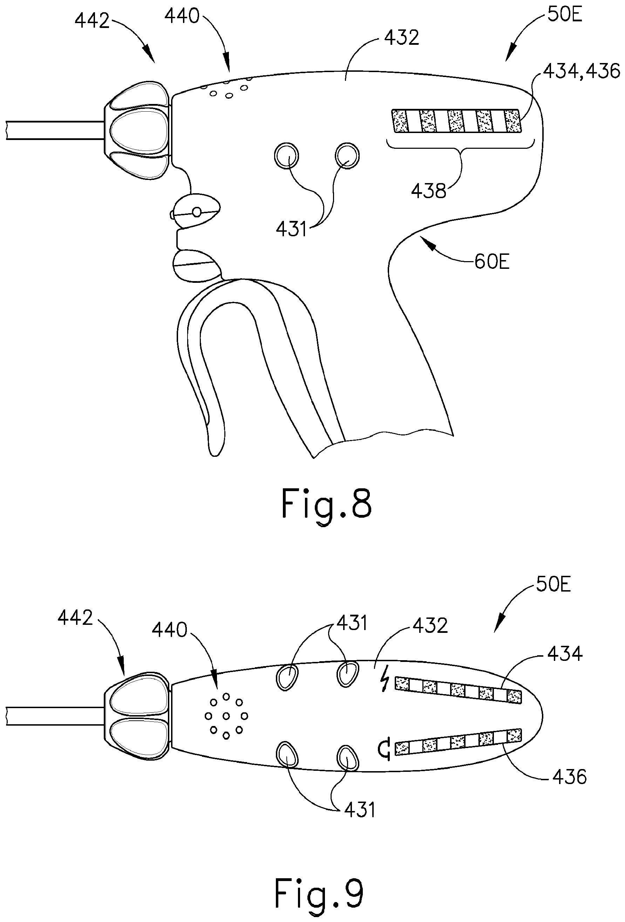

FIGS. 8-9, which are not in accordance with the present invention, show another version of exemplary ultrasonic surgical instrument (50E), which is similar to medical device (10, 100) described above. Surgical instrument (50E) includes hand activation buttons (431) and both auditory and visual feedback features. For example, as shown in FIG. 9, upper surface (432) of multi-piece handle assembly (60E) includes LED indicators (434, 436). LED indicator (434) may provide a visual display bar for indication of an amount of power left in surgical instrument (50E) by, for example, displaying an amount of power left in batteries power surgical instrument (50E). LED indicator (436) may provide a visual display bar for indication of measurements of impedance during use of instrument (50E). For instance, instrument (50E) of this example has an end effector (e.g., similar to end effector (140) described above) with jaws that include sensors. The sensors are used to measure the impedance of tissue being clamped by the jaws. This measured impedance may be monitored to determine the degree to which the tissue is sealed by ultrasonic energy or RF energy, etc. In addition or in the alternative, the measured impedance may be used as a feedback input through a control algorithm to control the delivery of ultrasonic energy or RF energy to the tissue via the jaws. Various suitable ways in which tissue impedance may be measured are described in various references cited herein.

LED indicator (436) as an impedance indicator may change by, for example, lighting up more LED bars or changing a color display as tissue impedance increases. Such a change allows a user to understand that instrument (50E) is approaching a back side of a power curve, or rather, that impedance or resistance to applied voltage has increased, indicating that portions of the operated upon tissue has been sufficiently sealed. Different patterns, such as pattern (438) shown in FIG. 8, may reflect codes relaying a message to a user. For example, if an error occurred when using instrument (50E), a beep pattern from a generator could change from a pre-defined normal pattern reflected by a pattern and number of lit LED bars to a different illuminated non-normal pattern to indicate the error. The normal and non-normal patterns may be set forth in a user guide, for example, to allow a user to interpret the appropriate error message and whether an error is occurring.

With respect to auditory feedback mechanisms, surgical instrument (50E) includes speaker holes (440) on distal end (442) of upper surface (432) of multi-piece handle assembly (60E). A speaker (not shown) disposed below speaker holes (440) will emit sounds to provide audible feedback during activation of instrument (50E). For example, a pattern of audible "beeps" may change upon selection of a minimum or maximum amount of activation power. Or, a pattern or tone may change relative to a measured amount of impedance or power left in the batteries powering surgical instrument (50E), such as projecting a lower pitch or tone as the batteries lose power.

### B. Exemplary Visual Indicators

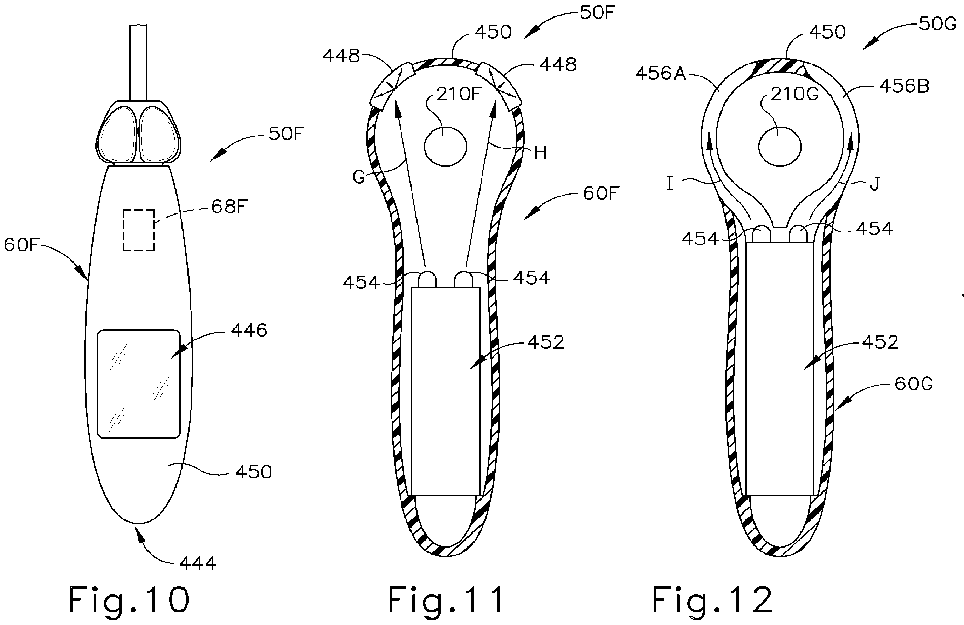

FIGS. 10-13 show an exemplary ultrasonic surgical instrument (50F), which is not in accordance with the present invention, and is similar to medical device (10, 100) described above, that additionally includes a visual indicator to provide visual feedback. FIG. 10 shows proximal end portion (444) of surgical instrument (50F) with a phantom view of projecting trigger (68F). Surgical instrument (50F) includes information display location (446), which may include, for example, a LCD screen including LED lighting imaging to reflect such information from instrument (50F) as described above for other instrument versions.

FIG. 11 shows a cross-section of multi-piece handle assembly (60F) of surgical instrument (50F) including acoustic waveguide (210F). Clear lens (448) is positioned on upper surface (450) of an exterior proximal end portion (444) either as part of information display location (446) or separate from information display location (446). Clear lens (448) may comprise plastic or any other suitable material. Cartridge (452) housed in multi-piece handle assembly (60F) includes electronic module(s) to transmit information and/or batteries to power surgical instrument (50F). One or more projecting light sources (454), which may comprise LEDs, are mounted on an upper surface of cartridge (452). In use, projecting light sources (454) project light through instrument (50F) in the respective directions of arrows (G, H) around acoustic assembly shown by acoustic waveguide (210F) through to clear lens (448), which collects the projected light and transmits the light to enable a user to see the light. Cartridge (452) is removable from multi-piece handle assembly (60F) of surgical instrument (50F) and includes one or more electronics modules. The electronics module(s) of cartridge (452) may control the display functions. Thus, cartridge (452) controls visual feedback to a user as cartridge (452) causes at least one projecting light source (454) to project a light that is seen by the user. The at least one projecting light source (454) is positioned on cartridge (452) such that it is visible to a user through at least one clear plastic lens (448) during use of instrument (50F).

It should be understood that the positioning of cartridge (452) and the use of lens (448) may permit assembly (60F) to be sterilized using gamma sterilization and/or other techniques without harming cartridge (452). In addition or in the alternative, cartridge (452) may be removed to undergo some other form of reprocessing while assembly (60F) is sterilized using gamma sterilization and/or other techniques. In other words, all of the electronic components of instrument (50F) may be contained on cartridge (452), such that the removability of cartridge (452) enables sterilization of assembly (60F) using techniques that might otherwise damage such electronic components.

It should also be understood that transparent bezels, light pipes, and light collectors may be used to assist with projecting light from light sources (454) through lens (448) on upper surface (450) of instrument (50F). For example, FIG. 12 shows an alternate version of exemplary ultrasonic surgical instrument (50G), similar to instrument (50F) described above. However, projecting light sources (454) on cartridge (452) project light in the directions of arrows (I, J) through light tunnels (456A, 456B), which may comprise light pipes or light fibers, for example.

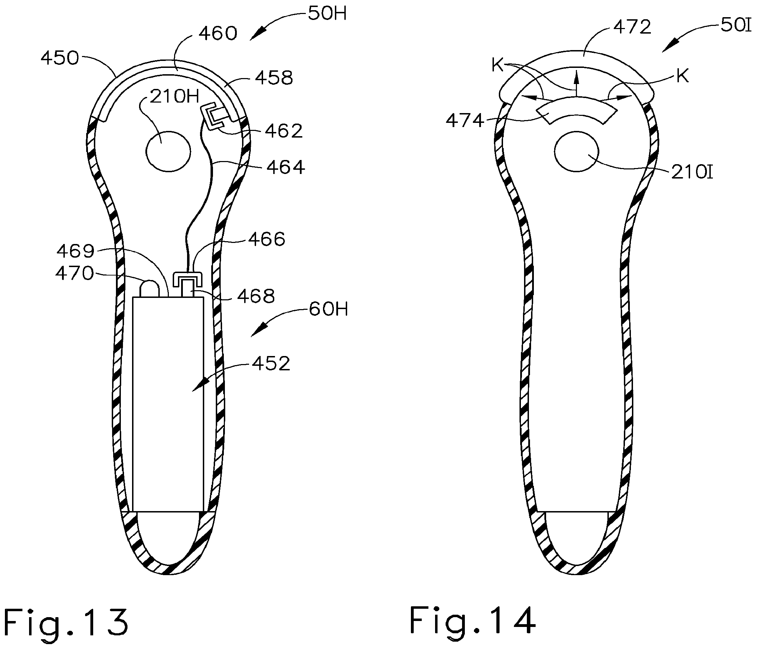

FIG. 13 shows an ultrasonic surgical instrument (50H), similar to medical device (10, 100) described above, with an exemplary alternative feature to provide visual feedback. Surgical instrument (50H) includes clear bezel (458) positioned on an exterior of upper surface (450) of instrument (50H). Flexible display (460) is disposed within multi-piece handle assembly (60H) or on upper surface (450) below clear bezel (458). Flexible display (460) conforms to the shape of upper surface (450) to better display information such as a display light, text, graphics, etc. through clear bezel (458). While flexible display (460) is shown as disposed beneath clear protective bezel (458), flexible display (460) may alternatively be disposed on the surface of instrument (50H) with a fluid tight electrical connection disposed within multi-piece handle assembly (60H) of instrument (50H). Flexible display (460) is connected to cartridge (452) via a feed-through contact shown as connector (462), discussed below, embedded in the shroud housing of multi-piece handle assembly (60H). Flexible display (460) is operable to display information such as light from light source (470), described below, may be driven by electronic connections from cartridge (452). Flexible display (460) may be a LED, organic LED ("OLED"), electro-chromic, active-matrix OLED ("AMOLED"), or backlight LCD type of display. Information from cartridge (452) is displayed on flexible display (460) for a user to see and use. A touch screen version may be used to enable control of settings such as generator settings through the display.

Connector (462) is in communication with cartridge (452). For example, wires (464) connect connector (462) to electrical connection (466). Electrical connection (466) is mated to a mating electrical connection (468) disposed on top surface (469) of cartridge (452) and electronically communicates with cartridge (452). While electrical connection (466) is shown to be a female connection mated to male mating electrical connection (468), the reverse mating orientation is possible. Light source (470) is disposed on top surface (469) of cartridge (452). Light source (470) may be a light for a back lit LCD or electro-chromic display.

FIG. 14 shows a surgical instrument (501), which is not in accordance with the present invention, and is similar to instrument (50H) described above but showing an alternative version. For example, surgical instrument (501) includes display surface or display screen (472) with projector lens (474) disposed below display screen (472). Projector lens (474) projects information, such as light, for example, onto display screen (472) in the direction of arrows (K). Thus, flexible display (460) is replaced by display screen (472), which provides a surface for projector lens (474) to project information to a user. Projector lens (474) is attached to cartridge (452) by a data cable (not shown) to allow projector lens (474) to project information to a user about instrument (501).

### III. Miscellaneous

Versions of the devices described above may have application in conventional medical treatments and procedures conducted by a medical professional, as well as application in robotic-assisted medical treatments and procedures. For instance, those of ordinary skill in the art will recognize that various teaching herein may be readily combined with various teachings of U.S. Pat. No. 6,783,524, entitled "Robotic Surgical Tool with Ultrasound Cauterizing and Cutting Instrument," published August 31, 2004.

Versions of described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a user immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various versions in the present disclosure, further adaptations of the methods and systems described herein may be accomplished by appropriate modifications by one of ordinary skill in the art without departing from the scope of the present claims. Several of such potential modifications have been mentioned, and others will be apparent to those skilled in the art. For instance, the examples, versions, geometries, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A surgical instrument comprising:
(a) a handpiece (60A), wherein the handpiece comprises a user feedback feature (408, 404, 416);
(b) a shaft assembly extending distally from the handpiece (60A); and
(c) an end effector (16, 140, 80A) disposed at a distal end of the shaft assembly, wherein the end effector is operable to delivery energy from the shaft assembly to a surgical site;
wherein the user feedback feature is operable to provide haptic feedback to the user, indicating information relating to operation of the end effector (16, 140, 80A) including confirmation of reaching a set threshold amount of resistance measured in ohms applied across operated upon tissue, and wherein the handpiece further comprises a user input feature (408), wherein the end effector includes an active feature responsive to actuation of the user input feature, and wherein the active feature is operable to operate on tissue in response to actuation of the user input feature, and **characterized in that** the information relating to operation of the end effector further includes confirmation of an activation of the end effector, and wherein the user input feature comprises a feedback pad (408) configured to receive one or more user inputted setting commands, and wherein the feedback pad (408) and the user feedback feature (404) form a single unit.

2. The surgical instrument of claim 1, wherein the user feedback feature (416) is operable to provide at least one of a visual or an auditory feedback and is operable to provide information relating to at least one of amplitude of a blade of the end effector or a power level associated with the surgical instrument.

3. The surgical instrument of claim 1, wherein the user feedback feature comprises a haptic motor configured to vibrate in response to a signal of a change in a status of the surgical instrument.

4. The surgical instrument of claim 1, wherein the user feedback feature comprises a visual indicator (416) operable to provide visual feedback to a user.

5. The surgical instrument of claim 4, wherein the visual indicator is configured to display information upon a screen (472) disposed on the surgical instrument in response to a signal of a change in a status of the surgical instrument.

6. The surgical instrument of claim 5, wherein the user input feature comprises a trigger (18, 68D), wherein the change in status is associated with a force applied to the trigger.

7. The surgical instrument of claim 5, further comprising a force sensor (426, 414), wherein the user input feature comprises a trigger (68D), wherein the force sensor (426, 414) is operable to measure force applied through the trigger.

8. The surgical instrument of claim 7, wherein the force sensor comprises a spring loaded gauge (414) in communication with the trigger (68D).

9. The surgical instrument of claim 7, wherein the force sensor is selected from the group consisting of a pressure sensor (426) in communication with the trigger (68D), a pressure sensor (426) in communication with the end effector, an acoustic impedance sensor (426), a tissue impedance sensor (426), and a displacement measuring device (426).

10. The surgical instrument of claim 5, wherein the displayed information comprises at least one of a pattern display or a bar display.

11. The surgical instrument of claim 5, further comprising a removable cartridge (452) disposed in the handpiece, wherein the screen (472) comprises clear plastic and is disposed on a proximal, upper surface of the handpiece, wherein the user feedback feature comprises one or more lighting devices (474) in communication with the cartridge (452), wherein the lighting devices (474) are operable to emit light through the clear plastic screen (472).

12. The surgical instrument of claim 5, wherein the user feedback feature further comprises a flexible display (460), wherein a cartridge (452) is in electrical communication with the flexible display (460), wherein the flexible display (460) is disposed below a clear plastic lens (458).

## Patentansprüche

1. Chirurgisches Instrument, umfassend:
(a) ein Handstück (60A), wobei das Handstück ein Benutzer-Feedbackelement (408, 404, 416) umfasst;
(b) eine Schaftanordnung, die sich distal vom Handstück (60A) erstreckt; und
(c) einen Endeffektor (16, 140, 80A), der an einem distalen Ende der Schaftanordnung angeordnet ist, wobei der Endeffektor dazu funktionsfähig ist, Energie von der Schaftanordnung an eine Operationsstelle zu liefern;
wobei das Benutzer-Feedbackelement dazu funktionsfähig ist, ein haptisches Feedback für den Benutzer bereitzustellen, das Informationen zum Betrieb des Endeffektors (16, 140, 80A) mit einer Bestätigung angibt, dass ein eingestellter Schwellenbetrag eines in Ohm gemessenen Widerstands erreicht ist, der an einem Gewebe anliegt, an dem operiert wird, und wobei das Handstück ferner ein Benutzer-Eingabeelement (408) umfasst, wobei der Endeffektor ein aktives Element aufweist, das auf die Betätigung des Benutzer-Eingabeelements reagiert und wobei das aktive Element dazu funktionsfähig ist, als Reaktion auf die Betätigung des Benutzer-Eingabeelements an Gewebe zu operieren, und **dadurch gekennzeichnet, dass** die Informationen über den Betrieb des Endeffektors ferner eine Bestätigung einer Aktivierung des Endeffektors enthalten, und
wobei das Benutzer-Eingabeelement ein Feedback-Pad (408) umfasst, das dazu konfiguriert ist, einen oder mehrere vom Benutzer eingegebene Einstellbefehle zu empfangen, und wobei das Feedback-Pad (408) und das Benutzer-Feedbackelement (404) eine einzige Einheit bilden.

2. Chirurgisches Instrument nach Anspruch 1, wobei das Benutzer-Feedbackelement (416) dazu funktionsfähig ist, mindestens eines von einem visuellen oder akustischen Feedback bereitzustellen, und dazu funktionsfähig ist, Informationen über mindestens eines von einer Amplitude einer Klinge des Endeffektors oder einem Leistungspegel des chirurgischen Instruments bereitzustellen.

3. Chirurgisches Instrument nach Anspruch 1, wobei das Benutzer-Feedbackelement einen Haptikmotor umfasst, der dazu konfiguriert ist, als Reaktion auf ein Signal einer Statusänderung des chirurgischen Instruments zu vibrieren.

4. Chirurgisches Instrument nach Anspruch 1, wobei das Benutzer-Feedbackelement eine visuelle Anzeige (416) umfasst, die dazu funktionsfähig ist, dem Benutzer ein visuelles Feedback zu geben.

5. Chirurgisches Instrument nach Anspruch 4, wobei die visuelle Anzeige dazu konfiguriert ist, als Reaktion auf ein Signal einer Statusänderung des chirurgischen Instruments Informationen auf einem Bildschirm (472) anzuzeigen, der am chirurgischen Instrument angeordnet ist.

6. Chirurgisches Instrument nach Anspruch 5, wobei das Benutzer-Eingabeelement einen Auslöser (18, 68D) umfasst, wobei die Statusänderung mit einer Kraft verbunden ist, die auf den Auslöser aufgebracht wird.

7. Chirurgisches Instrument nach Anspruch 5, ferner umfassend einen Kraftsensor (426, 414), wobei das Benutzer-Eingabeelement einen Auslöser (68D) umfasst, wobei der Kraftsensor (426, 414) dazu funktionsfähig ist, eine durch den Auslöser aufgebrachte Kraft zu messen.

8. Chirurgisches Instrument nach Anspruch 7, wobei der Kraftsensor einen federbelasteten Druckmesser (414) in Verbindung mit dem Auslöser (68D) umfasst.

9. Chirurgisches Instrument nach Anspruch 7, wobei der Kraftsensor aus der Gruppe ausgewählt ist, die aus einem Drucksensor (426) in Verbindung mit dem Auslöser (68D), einem Drucksensor (426) in Verbindung mit dem Endeffektor, einem Akustikimpedanzsensor (426), einem Gewebeimpedanzsensor (426) und einer Wegmessvorrichtung (426) besteht.

10. Chirurgisches Instrument nach Anspruch 5, wobei die angezeigten Informationen mindestens eines von einer Strukturanzeige oder einer Balkenanzeige umfassen.

11. Chirurgisches Instrument nach Anspruch 5, ferner umfassend eine abnehmbare Kassette (452), die im Handstück angeordnet ist, wobei der Bildschirm (472) einen transparenten Kunststoff umfasst und an einer proximalen oberen Fläche des Handstücks angeordnet ist, wobei das Benutzer-Feedbackelement eine oder mehrere Leuchtvorrichtungen (474) in Verbindung mit der Kassette (452) umfasst, wobei die Leuchtvorrichtungen (474) dazu funktionsfähig sind, Licht durch den transparenten Kunststoffbildschirm (472) zu emittieren.

12. Chirurgisches Instrument nach Anspruch 5, wobei das Benutzer-Feedbackelement ferner ein flexibles Display (460) umfasst, wobei eine Kassette (452) in elektrischer Verbindung mit dem flexiblen Display (460) steht, wobei das flexible Display (460) unter einer transparenten Kunststofflinse (458) angeordnet ist.

## Revendications

1. Instrument chirurgical comprenant :
(a) une pièce à main (60A), la pièce à main comprenant une caractéristique de rétroaction d'utilisateur (408, 404, 416) ;
(b) un ensemble de tige s'étendant de manière distale à partir de la pièce à main (60A) ; et
(c) un effecteur terminal (16, 140, 80A) disposé à une extrémité distale de l'ensemble de tige, l'effecteur terminal étant utilisable pour délivrer de l'énergie à partir de l'ensemble de tige à un site chirurgical ;
la caractéristique de rétroaction d'utilisateur étant utilisable pour fournir une rétroaction haptique à l'utilisateur, indiquant des informations relatives au fonctionnement de l'effecteur terminal (16, 140, 80A), comprenant la confirmation de l'atteinte d'une quantité seuil définie de résistance mesurée en ohms appliquée à travers le tissu opéré, et
la pièce à main comprenant en outre une caractéristique d'entrée d'utilisateur (408), l'effecteur terminal comprenant une caractéristique active sensible à l'actionnement de la caractéristique d'entrée d'utilisateur, et la caractéristique active étant utilisable pour agir sur le tissu en réponse à l'actionnement de la caractéristique d'entrée d'utilisateur, et
**caractérisé en ce que** les informations relatives au fonctionnement de l'effecteur terminal comprennent en outre la confirmation d'une activation de l'effecteur terminal, et
la caractéristique d'entrée d'utilisateur comprenant un pavé de retour (408) configuré pour recevoir une ou plusieurs commandes de réglage entrées par l'utilisateur, et le pavé de retour (408) et la caractéristique de rétroaction d'utilisateur (404) formant une seule unité.

2. Instrument chirurgical selon la revendication 1, la caractéristique de rétroaction d'utilisateur (416) étant utilisable pour fournir au moins une rétroaction visuelle ou auditive et étant utilisable pour fournir des informations relatives à au moins une amplitude d'une lame de l'effecteur terminal ou un niveau de puissance associé à l'instrument chirurgical.

3. Instrument chirurgical selon la revendication 1, la caractéristique de rétroaction d'utilisateur comprenant un moteur haptique configuré pour vibrer en réponse à un signal de changement d'état de l'instrument chirurgical.

4. Instrument chirurgical selon la revendication 1, la caractéristique de rétroaction d'utilisateur comprenant un indicateur visuel (416) utilisable pour fournir une rétroaction visuelle à un utilisateur.

5. Instrument chirurgical selon la revendication 4, l'indicateur visuel étant configuré pour afficher des informations sur un écran (472) disposé sur l'instrument chirurgical en réponse à un signal de changement d'état de l'instrument chirurgical.

6. Instrument chirurgical selon la revendication 5, la caractéristique d'entrée d'utilisateur comprenant un déclencheur (18, 68D), le changement d'état étant associé à une force appliquée au déclencheur.

7. Instrument chirurgical selon la revendication 5, comprenant en outre un capteur de force (426, 414), la caractéristique d'entrée d'utilisateur comprenant un déclencheur (68D), le capteur de force (426, 414) étant utilisable pour mesurer la force appliquée à travers le déclencheur.

8. Instrument chirurgical selon la revendication 7, le capteur de force comprenant une jauge à ressort (414) en communication avec le déclencheur (68D).

9. Instrument chirurgical selon la revendication 7, le capteur de force étant choisi dans le groupe constitué par un capteur de pression (426) en communication avec le déclencheur (68D), un capteur de pression (426) en communication avec l'effecteur terminal, un capteur d'impédance acoustique (426), un capteur d'impédance tissulaire (426) et un dispositif de mesure de déplacement (426).

10. Instrument chirurgical selon la revendication 5, les informations affichées comprenant au moins un affichage de motifs ou un affichage à barres.

11. Instrument chirurgical selon la revendication 5, comprenant en outre une cartouche amovible (452) disposée dans la pièce à main, l'écran (472) comprenant du plastique transparent et étant disposé sur une surface supérieure proximale de la pièce à main, la caractéristique de rétroaction d'utilisateur comprenant un ou plusieurs dispositifs d'éclairage (474) en communication avec la cartouche (452), les dispositifs d'éclairage (474) étant utilisables pour émettre de la lumière à travers l'écran en plastique transparent (472) .

12. Instrument chirurgical selon la revendication 5, la caractéristique de rétroaction d'utilisateur comprenant en outre un affichage flexible (460), une cartouche (452) étant en communication électrique avec l'affichage flexible (460), l'affichage flexible (460) étant disposé sous une lentille en plastique transparent (458).
